# EUROPÄISCHE PATENTSCHRIFT

(11) **EP 2 195 441 B1**
(45) Veröffentlichungstag und Bekanntmachung des Hinweises auf die Patenterteilung: **14.09.2011**
(21) Anmeldenummer: 08834523.6
(22) Anmeldetag: 24.09.2008
(51) Int. Cl.: C12P 7/56, C12P 7/62

(54) **Gewinnung von Milchsäure durch Fermentation und Extraktion mit alkylierten Aminen**
Production of lactic acid by fermentation and extraction using alkylated amines
Récupération d'acide lactique par fermentation et extraction à l'aide d'amines alkylées

(30) Priorität: 24.09.2007 DE 102007045701
(43) Veröffentlichungstag der Anmeldung: 16.06.2010
(73) Patentinhaber: Uhde GmbH, 44141 Dortmund (DE)
(72) Erfinder: SCHULZE, Joachim, 59494 Soest (DE); WASSERSCHEID, Peter, 91054 Erlangen (DE); BÖSMANN, Andreas, 91093 Heßdorf (DE); TIETZ, Wolfgang, 06408 Biendorf (DE)
(86) Internationale Anmeldenummer: PCT/EP2008/008057
(87) Internationale Veröffentlichungsnummer: WO 2009/040095

(56) Entgegenhaltungen:
- WO-A-01/25180
- WO-A-01/81610
- WO-A-92/05168
- WO-A-95/24496
- WO-A-99/19503
- WO-A-02/090312
- WO-A-2006/124899
- CHOUDHURY B ET AL: "STUDY OF LACTIC ACID EXTRACTION WITH HIGHER MOLECULAR WEIGHT ALIPHATIC AMINES" JOURNAL OF CHEMICAL TECHNOLOGY AND BIOTECHNOLOGY, BLACKWELL SCIENTIFIC PUBLICATIONS. OXFORD, GB, Bd. 72, Nr. 2, 1. Juni 1998 (1998-06-01), Seiten 111-116, XP000800229 ISSN: 0268-2575

## Beschreibung

Die Herstellung von Milchsäure aus kohlenhydrathaltigen Materialien durch Fermentation gewinnt zunehmend an Bedeutung. Milchsäure ist ein umweltfreundliches Zwischenprodukt zur Herstellung von Reinigern, Flüssigseifen, Entkalkem und Textilhilfsmitteln. Das Interesse an Milchsäure ist in der letzten Zeit weiter gewachsen, weil die polymere Form der Milchsäure, das Polylactid, kompostierbar ist. Polylactid oder Polymilchsäure findet Anwendung als biologisch abbaubarer und gut verträglicher Kunststoff in der Lebensmittelindustrie, in der Kosmetik und in der Medizintechnik. Besonderes Interesse finden Tragetaschen aus kompostierbaren Polymilchsäurefolien, weil Tragetaschen aus konventionellem Kunststoff in der Umwelt nicht abgebaut werden und deshalb eine große Umweltbelastung darstellen. Plastiktragetaschen aus Polymilchsäure sind hingegen biologisch abbaubar und deshalb eine umweltfreundliche Alternative zu Tragetaschen aus konventionellem Kunststoff.

Ausgangsstoff zur Herstellung von Milchsäure ist ein kohlenhydrathaltiges Material, das durch Umsetzung mit hierfür geeigneten Mikroorganismen in Milchsäure umgewandelt wird. Geeignete Bakterien hierfür sind beispielsweise Milchsäurebakterien vom Stamm der *Lactobacillaceae*, aber auch Mikroorganismen vom Stamm der *Saccharomyces* oder *Rhizopus*. Je nach eingesetztem Stamm der Mikroorganismen erhält man entweder das linksdrehende Enantiomer oder das rechtsdrehende Enantiomer der Milchsäure. So erhält man bei der Verwendung von *Lactobacillus bulgaris* ausschliesslich das linksdrehende Enantiomer, D-(-)-Milchsäure. Bei der Verwendung eines anderen Bakterienstammes, *Lactobacillus casei*, erhält man hingegen ausschließlich die andere enantiomere Form, L-(+)-Milchsäure.

Bei der fermentativen Umsetzung von Kohlenhydraten zu Milchsäure sinkt der pH-Wert der Fermentationslösung durch die Entstehung der Säure. Bei hoher Konzentration an Milchsäure kann der pH-Wert auf Werte bis zu 2,0 sinken. Liegt die Säure in äquimolarer Konzentration mit dem Salz vor, so beträgt der pH-Wert der Lösung 3,86, was dem pKₐ-Wert der Milchsäure (25°C) entspricht. Einige Bakterienstämme können bei niedrigem pH-Wert Milchsäure produzieren, die meisten Stämme jedoch benötigen zur Milchsäureproduktion einen höheren pH-Wert. Aus diesem Grund wird der Fermentationslösung bei der Herstellung der Milchsäure üblicherweise ein basisches Salz zugesetzt, das den pH-Wert auf Werte um 5,0 oder höher anhebt. Bei dem basischen Salz handelt es sich häufig um basische Calciumsalze, die mit der entstehenden Milchsäure schwerlösliche Calciumsalze bilden.

Das entstehende Calciumsalz kann aufgrund der Schwerlöslichkeit leicht aus dem Fermentationsprozess abgetrennt werden. Zur Gewinnung der Milchsäure wird das isolierte Calciumsalz dann mit einer Mineralsäure angesäuert, wobei die Milchsäure frei wird und das Calciumsalz der Mineralsäure anfällt. Bei Verwendung von Schwefelsäure als Mineralsäure erhält man schwerlösliches Calciumsulfat, das leicht durch Filtration aus dem Prozess entfernt werden kann.

Die Milchsäure kann aber auch durch Extraktion aus der Milchsäurelösung entfernt werden. Obwohl Milchsäure sehr hydrophil ist und deswegen nicht leicht aus einer wässrigen Lösung abgetrennt werden kann, gibt es einige Lösungsmittel, die zur Extraktion von Milchsäure aus einem wässrigen Medium geeignet sind. Besonders häufig für diesen Zweck verwendete Lösungsmittel sind Trialkylphosphate oder Trialkylphosphinoxide, die mit Wasser ein Zweiphasensystem bilden und Milchsäure aufgrund eines hinreichend großen Verteilungskoeffzienten gut lösen können. Häufig verwendete Lösungsmittel sind auch Tri-*n*-alkylamine, die Milchsäure aus Wasser extrahieren.

Es ist möglich, das Amin zur Extraktion eines Milchsäuresalzes zu verwenden, wobei als Milchsäuresalz bevorzugt ein Ammoniumlactat oder ein Alkylammoniumlactat eingesetzt wird. Die WO 2006/124633 A1 beschreibt einen Prozess zur Herstellung von Calciumlactat durch Fermentation, wobei das Calciumlactat durch Umsetzung mit Ammoniak oder einem Alkylamin und Kohlendioxid oder einem Äquivalent hierzu umgesalzt wird, so dass man ein Ammoniumlactat oder ein Alkylammoniumlactat erhält. Dieses wird mit einem organischen Lösungsmittel extrahiert, wonach der Extrakt erhitzt wird. Dabei wird die Milchsäure freigesetzt, die sich dann durch Extraktion mit Wasser oder durch Destillation reinigen läßt. Alternativ ist es möglich, statt von Calciumlactat von Ammoniumlactat auszugehen, wodurch der Umsalzungsschritt entfällt.

Auch die direkte Extraktion von Hydroxycarbonsäuren aus Fermentationsprozessen wurde bereits untersucht. Dies funktioniert, wenn ein Lösungsmittel verwendet wird, das nichttoxisch für die milchsäureproduzierenden Bakterienstämme ist und wenn der pH-Wert der Fermentationslösung auf einen genau einzustellenden pH-Wert eingestellt wird. Hano et al. berichten in der Veröffentlichung Bioseperation 3, 321-326, 1993 über eine Extraktion, die während der Fermentation bei einem pH-Wert von pH = 5 mit Di-*n*-octylamin als Lösungsmittel durchgeführt wird. Bei einer Durchführung der Extraktion nach der Fermentation kann man die Milchsäure mit einer Lösungsmittelkombination aus Di-*n*-octylamin und Hexan bei einem pH-Wert von pH = 2 bis 2,5 extrahieren. Zur Entfernung des Lösungsmittels aus der Milchsäure wird das Lösungsmittel vor der destillativen Abtrennung mit Ammoniak versehen.

Diese Prozesse bieten gegenüber der konventionellen Herstellung von Milchsäure über das Calciumlactat den Vorteil der einfacheren Handhabung. Da bei der Herstellung kein schwerlösliches Calciumsalz anfällt, das aufwendig von der Lösung durch Filtration getrennt und entsorgt werden muss, ist der apparative Aufwand erheblich geringer. Vorraussetzung ist, dass für die Extraktion und die Fermentation optimale pH-Werte gefunden werden und das ein geeignetes Lösungsmittel vorhanden ist, das nichttoxisch gegenüber den Milchsäure produzierenden Bakterienstämme ist, ein Zweiphasensystem mit Wasser bildet und auch eine genügend große Extraktionskraft für Milchsäure besitzt.

Obwohl eine extraktive Destillation technisch einfacher durchzuführen ist als eine Isolation über das Calciumsalz, besitzt sie den Nachteil, dass aus den genannten Gründen nicht immer eine geeignete Lösungsmittelkombination bereitsteht. Zudem kann es sein, dass das Lösungsmittel einen Teil der Kohlenhydrate mitextrahiert, was zu einer Verschlechterung der Ausbeute des gesamten Prozesses führt und die lsolierung des Produktes erschwert. Außerdem sind häufig mehrere Extraktionszyklen notwendig, um die Milchsäure vollständig aus dem wässrigen Fermentationsmedium abzutrennen. Für die Extraktion sind je nach Fermentationsprozess Lösungsmittelgermische notwendig, die je nach Phasenbildung aufwendig von der wässrigen Phase abzutrennen sind.

Die Schrift WO 95/24496 offenbart ein Verfahren zur Gewinnung von Milchsäure aus einer Lactat-Zufuhrlösung, indem in einem Extraktionsschritt die Lactat-Zufuhrlösung, die einen pH-Wert von 4 bis 14 hat, mit einem Extraktionsmittel kombiniert wird. Das Extraktionsmittel muss dabei ein mit Wasser nicht mischbares Trialklamin mit insgesamt mindestens 18 Kohlenstoffatomen sein. Dies geschieht in Gegenwart von Kohlendioxid bei einem Partialdruck von min 345 x 10³ Pa. Dabei bildet sich eine wässrige Phase und eine organische Phase, wobei letztere Phase die Milchsäure und das Extraktionsmittel enthält in einem letzten Schritt erfolgt die Abtrennung der Milchsäure aus der organischen Phase.

Es ist Aufgabe, ein kombiniertes Herstellungs- und Extraktionsverfahren für Milchsäure aus einem Fermentationsreaktor zur Verfügung zu stellen. Der pH-Wert der Fermentationslösung soll bei der Extraktion bevorzugt niedriger liegen als der pKₛ-Wert der Milchsäure. Die Abtrennung und Aufarbeitung des Extraktes sollen verfahrenstechnisch einfach sein und Milchsäure in hoher Reinheit und Ausbeute liefern. Das verwendete Lösungsmittel soll ohne Begleitkomponenten einsetzbar sein und mit Wasser ein Zweiphasengemisch bilden. Die erfindungsgemäße Milchsäuregewinnung soll zudem die leichte Integration eines Verfahrensschrittes ermöglichen, mit dem sich Oligooder Polylactide herstellen lassen.

Die Erfindung löst die Aufgabe durch einen Extraktionsprozess mit linearem *n*-Trioctylamin (TOA) oder einem anderen geeigneten Amin als Lösungsmittel. *n-*Trioctylamin besitzt im Phasendiagramm eine Mischungslücke mit Wasser, weshalb es bei Einhaltung eines bestimmten Mischungsverhältnisses leicht von diesem mit einer phasenabscheidenden Vorrichtung abtrennbar ist. Das aus Milchsäure gebildete Lactat ist ebenfalls flüssig, weist im Phasendiagramm mit Wasser eine Mischungslücke auf und ist ebenfalls leicht von Wasser und dem Ausgangsamin abtrennbar. Das Lactat lässt sich durch Wärmeeinwirkung leicht spalten, so dass man bei einer Destillation des gebildeten Ammoniumlactats leicht Milchsäure erhalten kann. Zusätzliche Lösungsmittel zur Einstellung der richtigen Lösungsmitteleigenschaft werden nicht benötigt.

Die Erfindung löst die Aufgabe insbesondere durch ein Verfahren zur Herstellung von Milchsäure aus kohlenhydrathaltigen Ausgangsmaterialien durch mindestens einen fermentativen Verfahrensschritt, und ist dadurch gekennzeichnet, dass
- ein kohlenhydrathaltiges Ausgangsmaterial in einem ersten Prozessschritt der Fermentation in einem Fermentationsreaktor, bei einer Temperatur von 20°C bis 60°C, einem pH-Wert von 2 - 4 und in Anwesenheit stickstoffhaltiger Nährmedien, durch Einwirkung von Mikroorganismen mit Ammoniak zu einer ammoniumlactathaltigen Lösung umgesetzt wird, und
- die so erhaltene ammonlumlactathaltige Lösung in einem nachfolgenden Prozessschritt der Extraktion mit einer Schwefelsäure oder Phosphorsäure und mit einem alkylierten Amin versehen wird, wobei der pH-Wert vor der Extraktion auf einen Wert unterhalb des pKₛ-Wertes der Milchsäure abgesenkt wird, und
- das so erhaltene Gemisch gut durchmischt oder gerührt wird, und dabei durch Extraktion eine dreiphasige Mischung erhalten wird, wobei die erste Phase überwiegend aus dem alkylierten Amin besteht, die zweite Phase überwiegend aus dem Salz des alkylierten Amins und der Milchsäure, und die dritte Phase überwiegend aus Wasser und Ammoniumsulfat besteht, und
- die dabei erhaltene dreiphasige Mischung in einer Vorrichtung zur Phasenabscheidung in drei Phasen aufgetrennt wird, und
- die erhaltene zweite Phase, die überwiegend aus dem Salz des alkylierten Amins und der Milchsäure besteht, destilliert wird, wobei man Milchsäure, das alkylierte Amin und einen schwersledenden Destillationsrückstand erhält, und
- die bei der Fermentation entstehenden biologischen Fermentationsrückstände entweder direkt nach der Fermentation, nach der Extraktion, bei der Phasenabscheidung oder bei der Destillation aus dem System entfernt werden.

Als kohlenhydrathaltige Ausgangsmaterialien für die Fermentation eignen sich insbesondere stärkehaltige Materialien wie Reis- oder Kartoffelstärke, Maischereste, Hydrolysate und Maisstärke. Geeignet sind aber auch zuckerhaltige Ausgangsstoffe, wie z.B. Dextrose, Saccharose, Glucose und Hexosen im Allgemeinen. Man kann aber auch kohlenhydrathaltige Materialien einsetzen, die aus Holzbestandteilen stammen, wie z.B Holzgummi, Xylose und Pentosen im Allgemeinen. Selbstverständlich kann man auch Gemische von Kohlenhydraten als Ausgangsmaterial für die fermentative Milchsäureherstellung einsetzen.

Als Mikroorganismen zur Herstellung von Milchsäure aus Kohlenhydraten sind insbesondere Stämme der Gattung *Lactobacillaceae* geeignet. Zur Ausführung des erfindungsgemäßen Verfahrens eignen sich ganz besonders Mikroorganismen aus der Gattung *Lactobacillus casei*. Geeignet sind aber auch Bakterienstämme der Gattung *Rhizopus*, *Pediococcus* oder *Saccharomyces*. Geeignet für die Ausführung des erfindungsgemäßen Verfahrens sind schließlich alle Mikroorganismen, die Kohlenhydrate in Milchsäure oder Lactate umwandeln können. Setzt man Stämme der Gattung *Lactobacillus* oder *Pediococcus* ein, so kann man die Fermentation bei moderaten pH-Werten von 5,0 bis 7,0 durchführen. Bevorzugt werden für das erfindungsgemäße Verfahren jedoch insbesondere Mikroorganismen, die bei niedrigen pH-Werten von 3,8 oder niedriger Milchsäure produzieren. Dies sind spezielle Mikroorganismen aus der Gattung *Lactobacillus*, wie sie beispielsweise in der EP 1025254 A1 genannt sind. In einer Ausführung der Erfindung kann auch eine Seed-Fermentation, die auch als Anzucht-Fermentation bezeichnet wird, durchgeführt werden.

Die eigentliche Fermentation wird beispielhaft batchweise in einer nährstoffhaltigen Lösung bei einer Temperatur von 20 °C bis 60 °C und einem pH-Wert von 4,0 bis 2,0 durchgeführt. Bevorzugt wird die Fermentation bei einer Temperatur von 25 °C bis 35 °C durchgeführt. Bevorzugt wird die Fermentation bei einem pH-Wert von 3,0 bis 2,5 durchgeführt. Zur Einstellung eines optimalen pH-Wertes wird die Fermentationslösung bei Bedarf mit einer Mineralsäure, bevorzugt mit Schwefelsäure oder Phosphorsäure versehen . Weiterhin können als Säure auch organische Carbonsäuren verwendet werden wie z.B. Acrylsäure und Bernsteinsäure. Zur Einstellung eines optimalen pH-Wertes wird die Fermentationslösung weiterhin mit Ammoniaklösung versehen. Diese kann, je nach Bindung der Milchsäure, auch nachdosiert werden, um den pH-Wert konstant zu halten.

Die Fermentationslösung gelangt nach der Fermentation in ein Extraktionsgefäß, wo sie mit *n*-Trioctylamin versetzt wird. Zur Freisetzung der Milchsäure wird das Ammoniumlactat mit einer Schwefelsäure oder Phosphorsäure versetzt. Nach der anschließenden Zugabe des *n*-Trioctylamins wird das Gemisch durch Rühren oder Schütteln innig gemischt. Dabei entsteht ein Drelphasengemisch. Die erste Phase besteht überwiegend aus reinem Tri-*n*-octylamin, die zweite Phase besteht überwiegend aus Tri-*n*-octylammoniumlactat und die dritte Phase besteht überwiegend aus wässriger Ammoniumsulfatlösung. Der pH-Wert sollte für die Erlangung einer optimalen Phasentrennung 4,0 bis 2,0 betragen, kann jedoch auch höher liegen. Die Temperatur wird in der Regel für den Zweck der Extraktion nicht geändert und kann bei 25 °C bis 35 °C liegen.

Das so erhaltene dreiphasige Gemisch gelangt dann in eine Vorrichtung zur Phasenabscheidung. Dort werden drei Phasen erhalten: Das überschüssige Tri-*n-*octylamin, das Tri-*n*-octylammoniumlactat und die wässrige Phase. Das Tri-*n*-octylamin wird in einer vorteilhaften Ausführung der Erfindung durch geeignete Leitungen und Vorrichtungen in den Prozess zurückgeführt Die wässrige Phase wird einer Entsorgung zugeführt. Sie enthält noch Ammoniumsulfat und maximal 2 Gew.-% Milchsäure. Diese kann durch geeignete Verfahren, wie z.B. mit einer Diffusionsmembran abgetrennt werden. Das Tri-*n*-octylammoniumlactat wird einer weiteren Aufarbeitung unterzogen.

Zur Erleichterung der Reaktionsführung können die festen Fermentationsrückstände, die während der Fermentation entstehen und im Wesentlichen aus Zellrückständen und festen Metaboliten bestehen, durch eine geeignete Vorrichtung abgetrennt werden. Dies geschieht vorteilhaft durch Filtrationseinrichtungen. Es können jedoch auch andere Trennvorrichtungen eingesetzt werden, die für die Abtrennung von festen Fermentationsrückständen geeignet sind. Die Vorrichtung zur Abtrennung der Fermentationsrückstände befindet sich in einer vorteilhaften Ausführung der Erfindung direkt hinter dem Fermentationsreaktor Es ist jedoch auch möglich, die Vorrichtung zur Abtrennung der festen Fermentationsrückstände hinter der Vorrichtung zur Extraktion oder hinter der Vorrichtung zur Aufarbeitung des Lactats zu integrieren. Die so erhaltenen Fermentationsrückstände fallen üblicherweise als Feststoffe an und werden einer Weiterverwendung oder Entsorgung zugeführt.

Es ist möglich, die hergestellte Milchsäure weiteren Verfahrensschritten zur Verbesserung der Qualität zu unterwerfen, wie beispielsweise einem Zusatz von entfärbenden Chemikalien. Dieser findet bevorzugt nach der Fermentation statt, kann jedoch an beliebiger Stelle im Prozessfluss erfolgen.

In einer Ausführung der Erfindung wird das aus der Phasentrennung erhaltene Tri-*n*-octylammoniumlactat einer weiteren Aufarbeitung unterzogen. Dies kann durch eine Vorrichtung zur Destillation geschehen. Aufgrund des Siedepunktes der Milchsäure (122 °C, 20 hPa) wird diese bevorzugt im Vakuum destilliert. Bei der Destillation wird das Tri-*n*-octylammoniumlactat rasch thermolysiert, so dass man Tri-*n-*octylamin und Milchsäure erhält. Die Milchsäure lässt sich dabei leicht in Reinform gewinnen. Als weiteres Produkt erhält man einen schwerlösliche und schwersiedenden Sumpf, der im Wesentlichen aus Tri-*n*-octylammoniumsulfat und Oligolactaten besteht.

In einer weiteren Ausführung der Erfindung wird das aus der Phasentrennung erhaltene Tri-*n*-octylammoniumlactat mit dem anfallenden überschüssigen Tri-n-octylamin zur Weiterverarbeitung erhitzt. Zur erfindungsgemäßen Ausführung dieses Teilschritts werden Temperaturen von 250 °C bis 350 °C angewendet. Dabei entsteht im Wesentlichen Tri-*n*-octylamin und ein Oligolactid, das im flüssigen Zustand anfällt und weiter destilliert werden kann. Die Temperaturen sind notwendig, um eine schnelle Einstellung des Gleichgewichtes zum Oligolactid zu erreichen. Vorteilhaft führt man die Thermolyse bei einer Temperatur von 300°C durch, um die Bildung des Oligolactids ausreichend schnell zu erreichen, aber eine Zersetzung der Stoffe zu verhindern.

Zur apparativen Durchführung der Thermolyse kann man auch einen Verdampfer einsetzen. Hierzu wird das Tri-*n*-octylaminlactat durch den Verdampfer geleitet, der mit Füllkörpern beschickt ist, die eine Zersetzung des Lactats ermöglichen. Solche Füllkörper bestehen vorzugsweise aus sauren Oxiden. Gut geeignet für diesen Zweck ist beispielsweise γ-Aluminiumoxid Auch andere veresterungsaktive Festkörper können eingesetzt werden. Prinzipiell kann jeder Füllkörper verwendet werden, der die Spaltung von Tri-*n*-octylaminiactat zu Amin und Milchsäure und die nachfolgende Bildung des Oligolactides ermöglicht.

Um eine Temperaturerhöhung für die Thermolyse zu ermöglichen, kann man bei der Thermolyse ein Inertgas durch den Verdampfer leiten. Als Inertgas kommen dabei Gase in Betracht, die nicht mit dem Lactat reagieren. Bevorzugt benutzt man als inertgas trockenes Argon. Es ist jedoch auch möglich, preiswerteren Stickstoff zu verwenden. Das entstehende Tri-*n*-octylamin kann wieder in den Prozess zurückgeführt werden. Bei der anschliessenden Destillation des Oligolactids im Hochvakuum erhält man reines Dilactid und einen schwerlöslichen Sumpf, der im Wesentlichen aus Tri-*n*-octylammoniumsulfat besteht. Das Diloctid kann rein isoliert werden.

Bei der Phasentrennung und der folgenden Destillation oder Thermolyse erhält man das extrahierende Amin zurück. Dieses wird zur Kostenreduktion in den Prozess zurückgeführt. Das Amin aus der Extraktion kann mit dem zurückerhaltenen Amin aus der Weiterverarbeitung vereinigt werden oder auf verschiedenen Wegen in den Prozess zurückgeführt werden. Je nach Reinheit kann das Amin vor der Zurückführung in den Prozess auch einem Reinigungsschritt unterzogen werden. Geeignete Reinigungsschritte sind eine erneute Destillation, eine erneute Filtration oder eine Reinigung durch ein Membranverfahren.

Zur Extraktion der Milchsäure können zur Ausführung des erfindungsgemäßen Verfahrens alle Amine verwendet werden, die eine hinreichend große Löslichkeit für die Milchsäure besitzen, die eine Mischungslücke mit Wasser aufweisen, und die ein Lactat bilden, das mit Wasser eine Mischungslücke aufweist. Das alkylierte Amin sollte eine Wasserlöslichkeit besitzen, die bei 25°C vorzugsweise weniger als 1 Massenprozent beträgt. Bevorzugt besitzt das für die Extraktion eingesetzte Amin jedoch eine Wasserlöslichkeit von weniger als 0,1 Massenprozent. Das Milchsäuresalz des Amins solite eine Wasserlöslichkeit besitzen, die ebenfalls vorzugsweise weniger als 1 Massenprozent beträgt. Bevorzugt besitzt das gebildete Ammoniumlactat eine Wasserlöslichkeit von weniger als 0,1 Massenprozent. Das gebildete Ammoniumlactat ist in der Regel flüssig. Damit ist es auch pumpbar. Die Förderung des Lactats findet deshalb bevorzugt durch Pumpen im flüssigen Zustand statt.

Geeignet sind für das Verfahren Amine, die primärer, sekundärer oder tertiärer Natur sein können. Bei den Substituenten des Amins handelt es sich bevorzugt um Kohlenwasserstoffreste. Als Kohlenwasserstoffreste gelten dabei beliebig konstituierte Substituenten wie z.B. Alkylreste, *iso*-Alkylreste, Cycloalkylreste, Arylreste oder Substituenten, die ihrerseits mit einem der genannten Substituenten substituiert sind, wie beispielsweise Arylalkylsubstituenten. Bevorzugt handelt es sich bei dem alkylierten Amin um ein solches, das in den Substituenten eine C-Zahl von insgesamt mehr als 10 Kohlenstoffatomen aufweist. Selbstverständlich ist es auch möglich, Kohlenwasserstoffreste zu verwenden, die mit Fremdsubstituenten versehen sind, wie beispielsweise Halogensubstituenten oder Nitrilsubstituenten. Bevorzugt wird zur Ausführung des erfindungsgemäßen Verfahrens jedoch Tri-*n*-octylamin verwendet.

Je nach eingesetztem Bakterienstamm ist es mit dem erfindungsgemäßen Verfahren auch möglich, reines L-(+)-Enantiomer oder reines D-(-)-Enantiomer zu erhalten. Je nach gewonnenem Enantiomer und nach optischer Reinheit des gewonnenen Enantiomerengemischs erhält man ein Milchsäureprodukt mit unterschiedlichen physikalischen Eigenschaften. So besitzt eine enantiomerenreine Milchsäure einen Schmelzpunkt von 53 °C, ein Racemat jedoch einen Schmelzpunkt von 16,8 °C.

Zur Ausführung des erfindungsgemäßen Verfahrens kann eine Vorrichtung eingesetzt werden, die sich zur Ausführung dieses Verfahrens eignet. Beansprucht wird insbesondere eine Vorrichtung, die dadurch gekennzeichnet ist, dass
- die Vorrichtung einen zur Fermentation geeigneten Reaktor umfasst, und
- sich an den Fermentationsreaktor ein Gefäß zur Extraktion anschliesst, und
- sich an das Gefäß zur Extraktion eine Vorrichtung zur Phasenabscheidung anschließt, die drei auftretende, nicht miteinander mischbare Phasen getrennt abführt, und
- sich an die Vorrichtung zur Phasenabscheidung eine Vorrichtung zur Thermolyse und Oligomerisierung anschließt, und
- sich an die Vorrichtung zur Phasenabscheidung eine Destillationskolonne anschließt.

Zur Durchführung der weiteren Verfahrensschritte ist es hilfreich, die bei der Fermentation gebildeten Fermentationsrückstande zu entfernen. Aus diesem Grund befindet sich in einer Ausführung der Erfindung hinter dem Fermentationsreaktor eine Vorrichtung zur Entfernung der Fermentationsrückstände. Dies können kontinuierüch oder batchweise arbeitende Einrichtungen sein.

Eine besonders geeignete Einrichtung hierfür ist ein cross-flow-Filter. Dieser Filter ermöglicht eine kontinuierliche und effektive Klärung der Fermentationsbrühe. Es ist jedoch auch möglich, zur Entfernung der Fermentationsrückstände eine Siebeinrichtung oder eine Zentrifuge zu verwenden. Beispiele für geeignete Zentrifugen zur Abtrennung der Fermentationsrückstände sind Dekantier- oder Röhrenzentrifugen. Je nach Ausführung der erfindungsgemäßen Vorrichtung kann die Einrichtung zur Entfernung der Fermentationsrückstände auch später im Prozessfluss angeordnet sein. Dies ist zum Beispiel der Fall, wenn die Entfernung der Fermentationsrückstände erst bei der Destillation des Tri-*n*-octylammoniumlactates erfolgen soll.

Zur praktischen Ausführung der Extraktion kann die Extraktion auch in einem Membranextraktor durchgeführt werden. Voraussetzung für die Verwendung dieser Einrichtung ist das Vorhandensein einer für Milchsäure permeablen Membran. Geeignete Membranen bestehen beispielsweise aus Polyethersulfonen oder Polytetrafluorethen. In diesem Fall verbleiben die Fermentationsrückstände auf der Fermentationsseite der Membran. Die Membranen werden auf der für Milchsäure durchlässigen Seite mit dem Amin umspült, damit sich das korrespondierende Ammoniumlactat bilden kann. Hinter dem Membranreaktor befinden sich vorteilhaft phasenabscheidende Einrichtungen, in denen das Amin von dem Ammoniumlactat abgetrennt und in den Membranreaktor zurückgeführt wird.

Zur Extraktion können Gefäße zur Anwendung kommen, die eine rasche Durchmischung der Fermentationsbrühe mit dem Amin erlauben. Diese Gefäße enthalten Vorrichtungen zum effektiven und kräftigen Rühren oder auch Vorrichtungen zum Schütteln des Gefäßes. Hinzu können geeignete Dosierungseinrichtungen kommen, mit denen man das Amin und die Schwefelsäure oder die Phosphorsäure zur Neutralisation zudosieren kann. In der Regel wird die Extraktion batchweise durchgeführt. Sie kann jedoch auch kontinuierlich durchgeführt werden. Nach der Extraktion erhält man ein diskontinuierliches Gemisch mit drei Phasen, bestehend aus dem Ammoniumlactat, dem Amin und der ammoniumsulfathaltigen wässrigen Phase.

Der Vorrichtung zur Extraktion schließen sich geeignete Vorrichtungen zur Phasentrennung an. Dort wird die wässrige Phase von der Aminphase und der Ammoniumlactatphase getrennt. Die wässrige Phase wird durch geeignete Vorrichtungen abgetrennt und der Entsorgung zugeführt. Geeignete Phasentrennvorrichtungen sind beispielsweise Dekantierer oder Schwerkraftabscheider.

Zur Destillation eignen sich Vorrichtungen, die zur Destillation des Phasengemischs Ammoniumlactat/Amin geeignet sind. Da die Milchsäure aufgrund ihres Siedepunktes (122 °C, 20 hPa) im Vakuum destilliert wird, enthält die Destillationsvorrichtung Vorrichtungen zur Aufrechterhaltung eines Vakuums. Beim Erhitzen des Phasengemischs aus Alkylammoniumlactat/Amin zersetzt sich das Alkylammoniumlactat, so dass im wesentlichen das Amin und die Milchsäure überdestillieren. Zur Verbesserung der Trennwirkung kann die Destillationseinrichtung mit Kolonnenaufsätzen, Glockenböden oder anderen trennwirkungsverbessernden Vorrichtungen ausgestattet sein. Als Produkt werden Milchsäure und das Alkylamin erhalten. Das nicht destillierbare Sumpfprodukt besteht im Wesentlichen aus Alkylammoniumsulfat und Oligotactaten und wird durch geeignete Einrichtungen entsorgt. Geeignet für eine destillative Reinigung der Milchsäure ist beispielhaft auch ein Destillationsverfahren mittels Dünnschichtverdampfung, wie es beispielhaft in der EP 1 232 137 B1 beschrieben wird.

Zur Thermolyse eignen sich Gefäße, die ein Erhitzen und eine effektive Durchmischung des Inhalts gestatten. In einer vorteilhaften Ausführung der Erfindung ist es jedoch auch möglich, einen Verdampfer zu verwenden, wie er beispielhaft in der WO 92/05168 A1 beschrieben wird. In einer vorteilhaften Ausführung der Erfindung enthält der Verdampfer Füllkörper, die aus einem sauren Oxid bestehen. Beispiele für saure Oxide sind γ-Aluminiumoxide. Geeignet sind aber auch Silica, Silica-Aluminiumoxid-Kombinationen oder Zeolithe.

Der Verdampfer kann auch eine Vorrichtung zum Durchleiten von Inertgas enthalten, mit dem sich das Amin durch den Verdampfer leiten lässt. Um das Amin von der Milchsäure abzutrennen, befindet sich hinter dem Verdampfer eine Destillationskolonne, mit dem sich das Amin von der gebildeten Milchsäure abdestillieren lässt. Vor der Destillationskolonne kann sich auch eine Einrichtung befinden, die eine Phasentrennung des Inhalts gestattet.

Um das Amin aus der Extraktion, aus der Thermolyse und aus der Destillation in den Prozess zurückzuführen, sind diese Vorrichtungen mit Vorrichtungen ausgerüstet, die eine Rückführung des Amins in den Prozess gestatten. Da das Lactat des eingesetzten Amins pumpbar ist, gehören hierzu auch die entsprechenden Rohrteitungen, Pumpen und Ventile. Hinzu können auch Vorrichtungen kommen, die eine Reinigung des Amins gestatten. Ein Beispiel ist eine Destillationskolonne, die auch trennwirkungserhöhende Einrichtungen enthalten kann, wie Glockenböden- oder Füllkörperkolonnen. Es ist auch möglich, zur Reinigung des Amins Filtrationseinrichtungen zu verwenden, oder jede andere, zur Reinigung des Amins geeignete Vorrichtungen. Beispielhaft verwendet werden können eine Precoat-Filtration, eine Ultrafiltration oder eine Simulated Moving Bed (SMB)-Filtration. Gegebenenfalls kann sich vor dieser Einrichtung noch ein Analyseninstrument zur Feststellung der Reinheit des Amins befinden, wie beispielsweise ein Instrument zur Messung des Brechungsindices.

Bei der Destillation des aus der Thermolyse erhaltenen Dilactids erhält man das Dilactid. Dieses wird durch geeignete Vorrichtungen der weiteren Verarbeitung zugeführt. Die bei dem Prozess gewonnene Milchsäure oder das Dilactid können für beliebige Zwecke verwendet werden. Es ist möglich, die Milchsäure als umweltfreundlichen Wirkstoff für Reinigungsmittel, Entkalker oder Kosmetika zu verwenden. Es ist aber auch möglich, die Milchsäure zur weiteren Umsetzung zu Chemikalien oder Polymilchsäure zu verwenden. Polymilchsäure ist ein gut einsetzbarer und verarbeitbarer Kunststoff, der gut biologisch abbaubar ist. Polymilchsäure lässt sich gut für Gebrauchsgegenstände des täglichen Bedarfs, für medizinische Instrumente und Implantate oder als Verpackungsmaterial nutzen. Besonders geeignet ist Polymilchsäure zur Herstellung von kompostierbaren Plastiktragetaschen. Die Herstellung von Polylactiden wird beispielhaft in der EP 1247808 A2 beschrieben.

Der beschriebene Prozess ist nicht nur zur Herstellung von Milchsäure geeignet, sondern generell zur Herstellung von α-Hydroxycarbonsäuren in großem Maßstab. Der erfindungsgemäße Prozess ermöglicht die kostengünstige Bereitstellung großer Mengen Milchsäure. Die Verwendung von Calciumsalzen mit anschließender Ansäuerung entfällt bei dem vorliegenden Prozess, so dass keine schwerlöslichen Calciumsalze zur Entsorgung anfallen. Die Extraktion ist mit geringem Aufwand durchzuführen und ermöglicht die einfache Reinigung von Milchsäure. Das verwendete Extraktionsmittel ist nichttoxisch gegenüber Mikroorganismen und lässt sich bei neutralen bis sauren pH-Werten, bevorzugt aber bei sauren pH-Werten verwenden. Je nach Umsatz des eingesetzten Mikrobenstammes erhält man die Milchsäure in hohen Ausbeuten. Die so gewonnene Milchsäure ist von hoher Reinheit und Qualität.

Die Ausbeute bei der Wiedergewinnung Amins liegt bei über 90 Prozent, so dass wenig Kosten für eingespeistes Amin während des Betriebes entstehen. Das Amin löst Kohlenhydrate nicht, so dass keine Verluste an Ausgangsmaterial während der Extraktion entstehen.

Die Erfindung beansprucht ausdrücklich die Herstellung von Milchsäure, .Bei Verwendung eines geeigneten Bakterienstammes kann man die Milchsäure mit dem erfindungsgemäßen Verfahren auch in enantiomerenreiner Form herstellen. Die Erfindung beansprucht ausdrücklich auch die Herstellung von enantiomerenreiner oder enantiomerenangereicherter Milchsäure,

Die Herstellung von Oligo- und Polylactiden aus der erfindungsgemäß hergestellten Milchsäure ist vorgeschem. Bei Verwendung nur eines der durch das erfindungsgemäße Verfahren hergestellten Milchsäureenantiomeren erhält man bei Herstellung der Oligo- oder Polylactide, die die entsprechenden stereochemischen Konfigurationen besitzen. Die Herstellung von Polymilchsäure-Copolymeren aus Milchsäure, die nach dem erfindungsgemäßen Verfahren gewonnen wurde, ist auch vorgeschen.

Die erfindungsgemäße Ausgestaltung zur Gewinnung von Milchsäure wird anhand von zwei Zeichnungen genauer erläutert, wobei das erfindungsgemäße Verfahren jedoch nicht auf diese Ausführungsformen beschränkt ist.

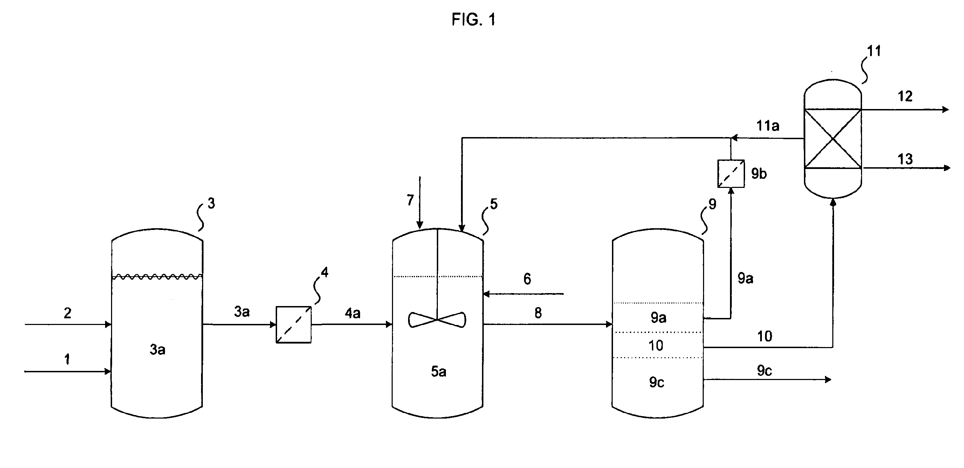

FIG. 1 zeigt eine erfindungsgemäße Vorrichtung zur Ausführung des erfindungsgemäßen Verfahrens, wobei die Milchsäure durch direkte Destillation des Ammoniumlactats gewonnen wird. Eine kohlenhydrathaltige wässrige Lösung (**1**) wird in einen Fermentationsbehälter (**3**) gegeben. Je nach Fortschreiten der Fermentation wird der pH-Wert durch Zugabe von Ammoniak (**2**) erhöht. Dabei entsteht eine ammoniumlactat-haltige Lösung (**3a**), die nach Abschluss der Fermentation in einen Extraktionsbehälter (**5**) gegeben wird. In der vorliegenden Ausführung wird die Fermentationsbrühe noch durch eine geeignete Vorrichtung (**4**) von den festen Fermentationstückständen befreit, so dass der Extraktionsbehälter mit bereits geklärter Lösung (**4a**) versetzt wird. Der Extraktionsbehälter ist mit geeigneten Zuführungsvorrichtungen versehen, über die die Fermentationsbrühe mit Schwefelsäure (**6**) zur Neutralisation versetzt wird. Über geeignete Zuführungsvorrichtungen wird auch das Amin (**7**) zur Extraktion zugegeben. Die Extraktionslösung (**6a**) wird dann zur Extraktion gerührt oder geschüttelt Man erhält dadurch eine Mischung (**8**) aus drei Phasen, die aus dem Amin, dem Ammoniumlactatsalz und einer wässrigen Phase besteht. Die Mischung wird in einen Behälter (**9**) gegeben, der zur Phasentrennung geeignet ist. Die wässrige, ammoniumsulfathaltige Phase (**9c**), die noch Spuren an Sulfatsalz des Amins enthält, wird ausgeschleust. Die aminhaltige Phase (**9a**) wird ebenfalls abgetrennt, gegebenenfalls durch eine Reinigungsvorrichtung gereinigt (**9b**) und anschließend in die Extraktion zurückgeführt. Die dritte Phase (**10**), die im Wesentlichen aus dem Lactat des Amins und noch geringeren Anteilen an Amin und dem Sulfatsalz des Lactats besteht, wird im Hochvakuum destilliert. Bei der Destillation (**11**) zersetzt sich das Salz aus dem Amin und der Milchsäure, so dass man reine Milchsäure (**12**) als Produkt erhält, die bei einer Temperatur von 122 °C (20 hPa) überdestilliert. Das aus der Destillation erhaltene Amin (**11a**) wird in die Extraktion zurückgeführt. Man erhält noch einen schwerlöslichen Destillationssumpf (**13**), der im Wesentlichen aus dem Sulfatsalz des Amins besteht. Die Milchsäure (**12**) wird dann der weiteren Verarbeitung zugeführt.

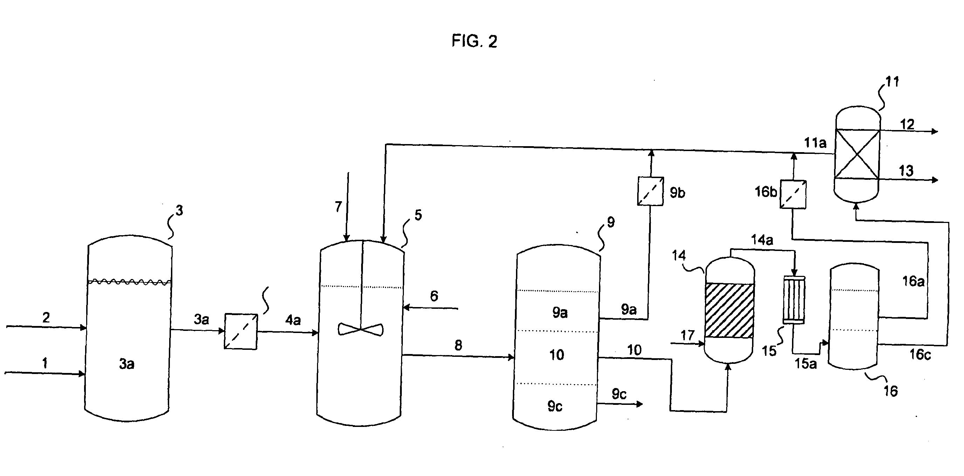

FIG. 2 zeigt eine erfindungsgemäße Vorrichtung zur Ausführung des erfindungsgemäßen Verfahrens, wobei durch Thermolyse des Oligolactides ein Dilactid gewonnen wird. Eine kohlenhydrathaltige wässrige Lösung (**1**) wird in einen Fermentationsbehälter (**3**) gegeben. Je nach Fortschreiten der Fermentation wird der pH-Wert durch Zugabe von Ammoniak (**2**) erhöht. Dabei entsteht eine ammoniumlactathaltige Lösung (**3a**), die nach Abschluss der Fermentation durch eine geeignete Vorrichtung (**4**) gereinigt wird und dadurch geklärt in einen Extraktionsbehälter (**5**) gelangt. Der Extraktionsbehälter ist mit geeigneten Zuführungsvorrichtungen versehen, über die die Fermentationsbrühe mit Phosphorsäure zur Neutralisation versetzt wird. Über geeignete Zuführungsvorrichtungen wird auch das Amin (**7**) zur Extraktion zugegeben. Die Extraktionslösung wird dann zur Extraktion gerührt oder geschüttelt. Man erhält dadurch eine Mischung aus drei Phasen, die aus dem Amin, dem Lactat des Amins und einer wässrigen Phase besteht. Die Mischung wird in einen Behälter (**9**) gegeben, der zur Phasentrennung geeignet ist. Die wässrige, ammoniumsulfathaltige Phase (**9c**), die noch Spuren des Sulfatsalzes des Amins enthält, wird ausgeschleust. Die aminhaltige Phase (**9a**) wird ebenfalls abgetrennt, gegebenenfalls durch eine Reinigungsvorrichtung (**9b**) gereinigt und anschließend in die Extraktion zurückgeführt. Die lactathaltige Phase (**10**), die im Wesentlichen das Lactatsalz des Amins umfasst, wird verdampft und in einen Verdampfer (**14**), der mit oxidischen Füllkörpern beaufschlagt ist, geleitet. Zur Unterstützung der Verdampfung kann dem verdampfenden Lactatstrom ein Inertgas (**17**) zugegeben werden. Dort wird das Lactatsalz des Amins verdampft und zersetzt. Aus der Verdampfung und Thermolyse (**14**) erhält man das gasförmige Amin und gasförmiges Oligolactat als Produkt (**14**a). Dieses Gemisch wird in einem Kondensator **(15)** kondensiert und das Kondensat (**15a**) in einen Phasenabscheider (**16**) gegeben. Dort erhält man das Amin (**16a**) zurück, das gegebenenfalls durch eine geeignete Vorrichtung (**16b**) gereinigt und mit dem Ausgangstrom einer erneuten Extraktion zugeführt wird. Das Oligolactat (**16c**) wird einer Destillation (**11**) im Hochvakuum zugeführt, in der man weiteres Amin (**11a**) und das reine Dilactid (**12**) erhält. Als weiteres Produkt fällt ein schwersiedender Destillationasumpf (**13**) an, der im Wesentlichen das Sulfatsalz des Amins umfasst. Das zusätzliche Amin (**11a**) kann in den Prozess zurückgeführt werden.

Bezugszeichenliste
- 1: Kohlenhydratlösung (in Wasser)
- 2: Wässrige Ammoniaklösung
- 3: Fermentationsbehälter
- 3a: Fermentationslösung
- 4: Reinigungsvorrichtung
- 4a: Geklärte Fermentationslösung
- 5: Extraktionsbehälter
- 6: Schwefelsäure oder Phosphorsäure
- 7: Alkyliertes Amin
- 8: Extraktionslösung
- 9: Phasenabscheider
- 9a: Phase mit alkyliertem Amin
- 9b: Reinigungsvorrichtung
- 9c: Wässrige Phase
- 10: Phase mit Lactatsatz des alkylierten Amins
- 11: Destillationseinheit
- 11a: Alkyliertes Amin
- 12: Milchsäure
- 13: Schwersiedender Destillationssumpf
- 14: Verdampfer
- 14a: Thermolysatgemisch (alkyliertes Amin und Oligalactid)
- 15: Kondensator
- 15a: Kondensat
- 16: Phasenabscheider
- 16a: Phase mit alkyliertem Amin
- 16b: Reinigungsvorrichtung
- 16c: Oligolactid
- 17: Inertgas

## Patentansprüche

1. Verfahren zur Herstellung von Milchsäure aus kohlenhydrathaltigen Ausgangsmaterialien durch mindestens einen fermentativen Verfahrensschritt, **dadurch gekennzeichnet, dass**
• ein kohlenhydrathaltiges Ausgangsmaterial in einem ersten Prozessschritt der Fermentation in einem Fermentationsreaktor, bei einer Temperatur von 20°C bis 60°C, einem pH-Wert von 2 - 4 und in Anwesenheit stickstoffhaltiger Nährmedien, durch Einwirkung von Mikroorganismen mit Ammoniak zu einer ammoniumlactathaltigen Lösung umgesetzt wird, und
• die so erhaltene ammoniumlactathaltige Lösung in einem nachfolgenden Prozessschritt der Extraktion mit einer Schwefelsäure oder Phosphorsäure und mit einem alkylierten Amin versehen wird, wobei der pH-Wert vor der Extraktion auf einen Wert unterhalb des pKₛ-Wertes der Milchsäure abgesenkt wird, und
• das so erhaltene Gemisch gut durchmischt oder gerührt wird, und dabei durch Extraktion eine dreiphasige Mischung erhalten wird, wobei die erste Phase überwiegend aus dem alkylierten Amin besteht, die zweite Phase überwiegend aus dem Salz des alkylierten Amins und der Milchsäure, und die dritte Phase überwiegend aus Wasser und Ammoniumsulfat besteht, und
• die dabei erhaltene dreiphasige Mischung in einer Vorrichtung zur Phasenabscheidung in drei Phasen aufgetrennt wird, und
• die erhaltene zweite Phase, die überwiegend aus dem Salz des alkylierten Amins und der Milchsäure besteht, destilliert wird, wobei man Milchsäure, das alkylierte Amin und einen schwersiedenden Destillationsrückstand erhält, und
• die bei der Fermentation entstehenden biologischen Fermentationsrückstände entweder direkt nach der Fermentation, nach der Extraktion, bei der Phasenabscheidung oder bei der Destillation aus dem System entfernt werden.

2. Verfahren nach Anspruch 1, **dadurch gekennzeichnet, dass** das bei der Phasentrennung erhaltene Amin in den Prozess der Extraktion zurückgeführt wird.

3. Verfahren nach einem der Ansprüche 1 bis 2, **dadurch gekennzeichnet, dass** das bei der der Extraktion nachgeschalteten Destillation anfallende alkylierte Amin in den Prozess der Extraktion zurückgeführt wird.

4. Verfahren nach einem der Ansprüche 1 bis 3, **dadurch gekennzeichnet, dass** es sich bei dem alkylierten Amin um ein Amin, das sich bei 25°C zu weniger als 1 Massenprozent in Wasser löst und dessen Milchsäuresalze sich ebenfalls zu weniger als 1 Massenprozent in Wasser lösen, handelt.

5. Verfahren nach einem der Ansprüche 1 bis 4, **dadurch gekennzeichnet, dass** es sich bei dem alkylierten Amin um ein primäres, sekundäres oder tertiäres Amin handelt.

6. Verfahren nach einem der Ansprüche 1 bis 5, **dadurch gekennzeichnet, dass** es sich bei dem alkylierten Amin um alkylierte Amine handelt, die in ihren Substituenten eine C-Zahl von insgesamt mehr als 10 Kohlenstoffatomen aufweisen.

7. Verfahren nach einem der Ansprüche 1 bis 6, **dadurch gekennzeichnet, dass** es sich bei den Substituenten des alkylierten Amins um Alkyl-, *iso*-Alkyl-, Cycloalkyl-, Aryl- oder Arylalkylsubstituenten handelt.

8. Verfahren nach einem der Ansprüche 1 bis 3, **dadurch gekennzeichnet, dass** es sich bei dem alkylierten Amin um Trioctylamin handelt.

9. Verfahren nach Anspruch 1, **dadurch gekennzeichnet, dass** der pH-Wert vor der Extraktion auf einen Wert kleiner als 3 abgesenkt wird.

10. Verfahren nach einem der Ansprüche 1 bis 9, **dadurch gekennzeichnet, dass** die nach der Extraktion erhaltene lactathaltige Phase erhitzt wird, wobei man durch Thermolyse und Oligomerisierung ein Oligolactid und das alkylierte Amin zurückerhält und die Thermolyse und Oligomerisierung bei einer Temperatur von 250 °C bis 350 °C stattfindet.

11. Verfahren nach Anspruch 10, **dadurch gekennzeichnet, dass** die nach der Extraktion erhaltene lactathaltige Phase zur Herstellung des Oligolactids beim Erhitzen mit einem Inertgas durchströmt wird.

12. Verfahren nach Anspruch 11, **dadurch gekennzeichnet, dass** das Inertgas Argon oder Stickstoff enthält.

13. Verfahren nach einem der Ansprüche 10 bis 12, dass das bei der Thermolyse entstehende alkylierte Amin in den Prozess der Extraktion zurückgeführt wird.

14. Verfahren nach einem der Ansprüche 1 bis 13, **dadurch gekennzeichnet, dass** bei der Herstellung der Milchsäure stereoselektiv das L-(+)-Enantiomer oder das D-(-)-Enantiomer produziert wird.

15. Verfahren nach einem der Ansprüche 10 bis 14, dass das bei der Thermolyse und Oligomerisierung entstehende Oligolactid nach Erhalt destilliert wird, wobei reines Dilactid erhalten wird.

16. Verfahren nach Anspruch 1, **dadurch gekennzeichnet, dass** der Prozessschritt der Extraktion der lactathaltigen Lösung in einem Membranreaktor durchgeführt wird, der eine für Milchsäure permeable Membran besitzt.

17. Verfahren nach Anspruch 1, **dadurch gekennzeichnet, dass** die erhaltene Milchsäure direkt nach der Destillation in der Dampfphase über einem geeigneten Katalysator zu einem Lactid umgesetzt wird.

18. Verfahren nach Anspruch 1, **dadurch gekennzeichnet, dass** als Ausgangsmaterial für die fermentative Milchsäureherstellung Saccharose, ein Gemisch aus Hexosen oder ein Gemisch aus Hexosen und Pentosen oder ein Gemisch dieser Kohlenhydrate verwendet wird.

19. Verfahren nach Anspruch 1, **dadurch gekennzeichnet, dass** als Mikroorganismen für die Milchsäureherstellung hierfür geeignete Bakterienstämme eingesetzt werden und bevorzugt die Gattungen der Lactobacillaceae, Pediococcus, Saccharomyces oder Rhizopus eingesetzt werden..

20. Verfahren nach Anspruch 1, **dadurch gekennzeichnet, dass** das aus der Fermentation kommende Produkt mit geeigneten Chemikalien zur Entfärbung versetzt wird.

21. Vorrichtung zur Ausführung eines Verfahrens nach einem der Ansprüche 1 bis 20,
**dadurch gekennzeichnet, dass**
• die Vorrichtung einen zur Fermentation geeigneten Reaktor umfasst, und
• sich an den Fermentationsreaktor ein Gefäß zur Extraktion anschliesst, und
• sich an das Gefäß zur Extraktion eine Vorrichtung zur Phasenabscheidung anschließt, die drei auftretende, nicht miteinander mischbare Phasen getrennt abführt, und
• sich an die Vorrichtung zur Phasenabscheldung eine Vorrichtung zur Thermolyse und Oligomerisierung anschließt, und
• sich an die Vorrichtung zur Phasenabscheidung eine Destillationskolonne anschließt.

22. Vorrichtung nach Anspruch 21, **dadurch gekennzeichnet, dass** sich zwischen dem Fermentationsreaktor und dem Gefäß zur Extraktion eine Vorrichtung zur Entfernung der biologischen Fermentationsrückstände befindet.

23. Vorrichtung nach Anspruch 22, **dadurch gekennzeichnet, dass** die Vorrichtung zur Filtration der Fermentationsrückstände eine Precoat-Fitration, eine Ultrafiltration oder eine Simulated-Moving-Bed-Filtration ist.

24. Vorrichtung nach Anspruch 23, **dadurch gekennzeichnet, dass** die sich in dem Extraktionsgefäß befindende Flüssigkeit gerührt werden kann.

25. Verfahren nach Anspruch 21 bis 24, **dadurch gekennzeichnet, dass** die Vorrichtung zur Destillation Vorrichtungen zur Aufrechterhaltung eines Vakuums enthält.

26. Vorrichtung nach Anspruch 21, **dadurch gekennzeichnet, dass** die Vorrichtung zur Thermolyse und Oligomerisierung einen Verdampfer mit Füllkörpern umfasst.

27. Vorrichtung nach Anspruch 26, **dadurch gekennzeichnet, dass** der Füllkörper γ-Aluminiumoxid enthält.

## Claims

1. Process for the production of lactic acid from carbohydrate-bearing feedstocks by means of at least one fermentative process step,
**characterised in that**
• a carbohydrate-bearing feedstock is converted to form an ammonium lactate-bearing solution through the action of micro-organisms and ammonia in a first process step of the fermentation in a fermentation reactor at a temperature of 20°C to 60°C and a pH value of 2 - 4 in the presence of nitrogen-containing culture medium, and
• a sulphuric acid or phosphoric acid and an alkylated amine are added to the ammonium lactate-bearing solution thus obtained in a subsequent process step, the pH value being lowered to a value lower than the pKₛ value of the lactic acid prior to extraction, and
• the mixture thus produced is thoroughly mixed or stirred, thereby obtaining through extraction a three-phase mixture, the first phase mainly consisting of the alkylated amine, the second phase mainly of the salt of the akylated amine and the third mainly of water and ammonium sulphate, and
• the three-phase mixture thus obtained is split into three phases in a device for phase separation, and
• the second phase obtained, which primarily consists of the salt of the alkylated amine and the lactic acid, undergoes distillation, which yields lactic acid, the alkylated amine and a high-boiling distillation residue, and
• the biological fermentation residues obtained during the fermentation are removed from the system either directly after the fermentation, after the extraction, during phase separation or during distillation.

2. Process according to Claim 1, **characterised in that** the amine obtained during phase separation is recycled to the extraction process.

3. Process according to either of claims 1 or 2, **characterised in that** the alkylated amine accumulated during the distillation downstream of the extraction is recycled to the extraction process.

4. Process according to one of claims 1 to 3, **characterised in that** the alkylated amine is an amine that is less than one percent water soluble by mass at 25°C and whose lactic acid salts are likewise less than one percent water soluble by mass.

5. Process according to one of claims 1 to 4, **characterised in that** the alkylated amine is a primary, secondary or tertiary amine.

6. Process according to one of claims 1 to 5, **characterised in that** the alkylated amine is one that totals a C-number of more than 10 carbon atoms in its substituents.

7. Process according to one of claims 1 to 6, **characterised in that** the substituents of the alkylated amine are alkyl, *iso*-alkyl, cycloalkyl, aryl or arylalkyl substituents.

8. Process according to one of claims 1 to 3, **characterised in that** the alkylated amine is trioctylamine.

9. Process according to claim 1, **characterised in that** the pH value is reduced to a value of less than 3 prior to extraction.

10. Process according to one of claims 1 to 9, **characterised in that** the lactate-bearing phase obtained after the extraction is heated, thereby retrieving an oligolactide and the alkylated amine by way of thermolysis and oligomerisation, said thermolysis and oligomerisation taking place at a temperature of 250°C to 350°C.

11. Process according to claim 10, **characterised in that** the lactate-bearing phase obtained after the extraction is permeated by an inert gas during heating so as to produce oligolactide.

12. Process according to claim 11, **characterised in that** the inert gas contains argon or nitrogen.

13. Process according to one of claims 9 to 12, **characterised in that** the alkylated amine obtained by way of thermolysis is recycled to the extraction process.

14. Process according to one of claims 1 to 13, **characterised in that** the production of lactic acid stereoselectively yields the L-(+) enantiomer or the D-(-) enantiomer.

15. Process according to one of claims 9 to 14, **characterised in that** the oligolactide obtained by way of thermolysis and oligomerisation subsequently undergoes distillation, thereby producing pure dilactide.

16. Process according to claim 1, **characterised in that** the process step of extracting the lactate-bearing solution is carried out in a membrane reactor equipped with a membrane permeable to lactic acid.

17. Process according to claim 1, **characterised in that** the lactic acid obtained is converted to lactide in the vaporous phase over a suitable catalyst directly after distillation.

18. Process according to claim 1, **characterised in that** the feedstock for the fermentative production of lactic acid is saccharose, a mixture of hexoses or a mixture of hexoses and pentoses, or a mixture of these carbohydrates.

19. Process according to claim 1, **characterised in that** suitable strains of bacteria are used as micro-organisms for the production of lactic acid, these preferably being of the lactobacillaceae, pediococcus, saccharomyces or rhizopus genus.

20. Process according to claim 1, **characterised in that** the product yielded by the fermentation is mixed with suitable chemicals for decolourisation.

21. Device for carrying out a process according to one of claims 1 to 20, **characterised in that**
• the device includes a reactor suitable for fermentation processes, and
• an extraction vessel is arranged downstream of the fermentation reactor, and
• a device for phase separation is installed downstream of the extraction vessel, with said device separately discharging the three non-miscible phases that occur,
• a device for thermolysis and oligomerisation is integrated downstream of the device for phase separation, and
• a distillation column is arranged downstream of the device for phase separation.

22. Device according to claim 21, **characterised in that** a device for the removal of biological fermentation residues is integrated between the fermentation reactor and the extraction vessel.

23. Device according to claim 22, **characterised in that** the device for filtering the fermentation residues is a precoat filtration unit, an ultrafiltration unit or a simulated moving-bed filtration unit.

24. Device according to claim 23, **characterised in that** the liquid in the extraction vessel can be stirred.

25. Process according to claims 21 to 24, **characterised in that** the distillation device is equipped with gadgets for maintaining a vacuum.

26. Device according to claim 21, **characterised in that** the device for thermolysis and oligomerisation encompasses a packed evaporator.

27. Device according to claim 26, **characterised in that** the packing contains γ-aluminium oxide.

## Revendications

1. Procédé de fabrication d'acide lactique à partir de substances de base contenant des hydrates de carbone par au moins une étape de fermentation,
**caractérisé en ce que**
• une substance de base contenant des hydrates de carbone est convertie en une réaction contenant du lactate d'ammonium lors d'une première étape de fermentation dans un réacteur à fermentation, à une température comprise entre 20°C et 60°C, avec un pH de 2 à 4 et en présence de substrats azotés, par action de microorganismes avec l'ammoniac et que
• à la réaction contenant du lactate d'ammonium ainsi obtenue sont ajoutés lors d'une étape suivante d'extraction un acide sulfurique ou phosphorique et une amine alkylée, en faisant abaisser avant l'extraction le pH en-dessous du pKₛ de l'acide lactique et que
• le mélange ainsi obtenu est bien mélangé ou agité pour obtenir par extraction un mélange triphasé, la première phase se composant principalement de l'amine alkylée, la deuxième phase se composant principalement du sel de l'amine alkylée et de l'acide lactique et la troisième phase se composant principalement d'eau et de sulfate d'ammonium et que
• le mélange triphasé ainsi obtenu est séparé en trois phases dans un dispositif de précipitation de phases et que
• la deuxième phase obtenue, se composant principalement du sel de l'amine alkylée et de l'acide lactique, est distillée en obtenant de l'acide lactique, l'amine alkylée ainsi qu'un résidu de distillation à faible point d'ébullition et que
• les résidus biologiques formés lors de la fermentation sont retirés du système soit directement après la fermentation ou après l'extraction, ou bien pendant la précipitation des phases ou la distillation.

2. Procédé selon la revendication 1, **caractérisé en ce que** l'amine obtenue lors de la séparation des phases est réintroduite dans le process d'extraction.

3. Procédé selon une des revendications 1 à 2, **caractérisé en ce que** l'amine alkylée résultant de la distillation effectuée en aval de l'extraction est réintroduite dans le process d'extraction.

4. Procédé selon une des revendications 1 à 3, **caractérisé en ce que** l'amine alkylée est telle qu'à 25°C elle se dissout dans l'eau à moins d'1 pourcentage de masse et que le sel de l'acide lactique se dissout dans l'eau également à moins d'1 pourcentage de masse.

5. Procédé selon une des revendications 1 à 4, **caractérisé en ce que** l'amine alkylée est une amine primaire, secondaire ou tertiaire.

6. Procédé selon une des revendications 1 à 5, **caractérisé en ce que** les amines alkylées présentent dans leurs substituants un nombre total de C supérieur à 10 atomes de carbone.

7. Procédé selon une des revendications 1 à 6, **caractérisé en ce que** les substituants de l'amine alkylée sont des substituants alkyles, *iso*-alkyles, cyclo-alkyles, aryles ou arylalkyles.

8. Procédé selon une des revendications 1 à 3, **caractérisé en ce que** l'amine alkylée est un trioctylamine.

9. Procédé selon la revendication 1, **caractérisé en ce que** le pH est abaissé à une valeur inférieure à 3 avant l'extraction.

10. Procédé selon une des revendications 1 à 9, **caractérisé en ce que** la phase lactatée obtenue après extraction est chauffée, en obtenant en retour par thermolyse et oligomérisation un oligolactide et l'amine alkylée, la thermolyse et l'oligomérisation étant réalisées à une température comprise entre 250 °C et 350 °C.

11. Procédé selon la revendication 10, **caractérisé en ce que** la phase lactatée obtenue par extraction est parcourue lors du réchauffage par un gaz inerte pour obtenir l'oligolactide.

12. Procédé selon la revendication 11, **caractérisé en ce que** le gaz inerte contient de l'argon ou de l'azote.

13. Procédé selon une des revendications 9 à 12, **caractérisé en ce que** l'amine alkylée formée lors de la thermolyse est réintroduite dans le process d'extraction.

14. Procédé selon une des revendications 1 à 13, **caractérisé en ce que** lors de la fabrication de l'acide lactique est produit de manière stéréosélective l'énantiomère L(+) ou l'énantiomère D(-).

15. Procédé selon une des revendications 9 à 14, **caractérisé en ce que** l'oligolactide formé lors de la thermolyse et de l'oligomérisation est distillé après obtention, en obtenant du dilactide pur.

16. Procédé selon la revendication 1, **caractérisé en ce que** l'étape d'extraction de la solution lactatée est réalisée dans un réacteur à membranes équipé d'une membrane perméable pour acide lactique.

17. Procédé selon la revendication 1, **caractérisé en ce que** l'acide lactique obtenu est converti en lactide directement après la distillation dans la phase vapeur au moyen d'un catalyseur approprié.

18. Procédé selon la revendication 1, **caractérisé en ce que** la substance de base utilisé pour la fabrication d'acide lactique enzymatique est du saccharose, un mélange d'hexoses et de pentoses ou un mélange de ces hydrates de carbone.

19. Procédé selon la revendication 1, **caractérisé en ce que** des souches bactériennes adéquates sont utilisées comme microorganismes pour la fabrication d'acide lactique et que sont utilisées de préférence les familles des lactobacillaceaes, pediococcus, saccharomyces ou rhizopus.

20. Procédé selon la revendication 1, **caractérisé en ce que** le produit né de la fermentation est mélangé à des produits chimiques adéquats pour être décoloré.

21. Dispositif pour exécuter un procédé selon une des revendications 1 à 20,
**caractérisé en ce que**
• le dispositif comprend un réacteur adapté à la fermentation et que
• un vase se raccorde au réacteur de fermentation pour l'extraction et que
• un dispositif de précipitation de phases se raccorde au vase d'extraction, permettant d'évacuer séparément les trois phases obtenues, non mélangeables entre elles et que
• un dispositif de thermolyse et d'oligomérisation se raccorde au dispositif de précipitation de phases et que
• une colonne de distillation se raccorde au dispositif de précipitation de phases.

22. Dispositif selon la revendication 21, **caractérisé par** la présence d'un dispositif d'élimination des résidus biologiques de fermentation, placé entre le réacteur de fermentation et le vase d'extraction.

23. Dispositif selon la revendication 22, **caractérisé en ce que** le dispositif de filtration des résidus de fermentation est une filtration précoat, une ultrafiltration ou une filtration par lit mobile simulé.

24. Dispositif selon la revendication 23, **caractérisé en ce que** le liquide présent dans le vase d'extraction peut être agité.

25. Dispositif selon la revendication 21 à 24, **caractérisé en ce que** le dispositif de distillation contient des dispositifs de maintien du vide.

26. Dispositif selon la revendication 21, **caractérisé en ce que** le dispositif de thermolyse et d'oligomérisation comprend un évaporateur avec éléments de remplissage.

27. Dispositif selon la revendication 26, **caractérisé en ce que** l'élément de remplissage contient de l'oxyde d'aluminium γ.
